# EUROPEAN PATENT APPLICATION

(11) **EP 4 279 488 A1**
(43) Date of publication of application: **22.11.2023**
(21) Application number: 22739190.1
(22) Date of filing: 18.01.2022
(51) Int. Cl.: C07D 403/12, A61K 31/506

(54) **SYNTHESIS METHOD FOR AMINOPYRIMIDINE FAK INHIBITOR COMPOUND**

(30) Priority: 18.01.2021 CN 202110063534
(71) Applicant: Hinova Pharmaceuticals Inc., Chengdu, Sichuan 610041 (CN)
(72) Inventor: DU, Wu, Chengdu, Sichuan 610041 (CN); LV, Haibin, Chengdu, Sichuan 610041 (CN); LI, Yu, Chengdu, Sichuan 610041 (CN); KUANG, Tongtao, Chengdu, Sichuan 610041 (CN); GENG, Xi, Chengdu, Sichuan 610041 (CN)
(74) Representative: Altmann Stößel Dick Patentanwälte PartG mbB
(86) International application number: PCT/CN2022/072552
(87) International publication number: WO 2022/152315

(57) **Abstract**

A synthesis method for an aminopyrimidine FAK inhibitor compound, relating to the field of pharmaceutical synthesis. In the synthesis method, R¹ is hydrogen or a carboxylic acid protecting group, R² is selected from C₁-C₆ alkyl or C₁-C₆ deuterated alkyl, R³ and R⁴ are each independently selected from hydrogen or deuterium, and R⁵ is selected from C₁-C₆ alkyl or C₁-C₆ deuterated alkyl. The synthesis method is simple to operate, and has low costs. The product prepared can obtain a high total yield (≥ 66%) and a high purity (≥ 99%), and the product yield and purity are superior to those in the prior art (in the prior art, the total yield is about 11%, and the purity is 99%). The synthesis method achieves excellent effects, can also successfully prepare a final product on kilogram scale, and is suitable for industrial production, having a good application prospect.

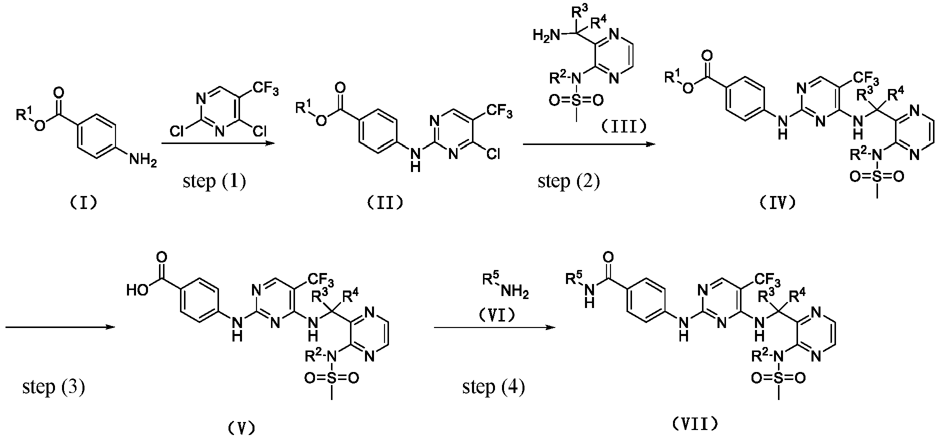

## Description

### Technical field

The present invention relates to the field of pharmaceutical synthesis, and in particular to a method for synthesizing aminopyrimidine FAK inhibitor compounds.

### Background technology

Defactinib (VS-6063) is a selective and oral FAK inhibitor developed by Verastem, with a structural formula 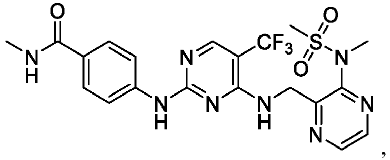 and is currently undergoing clinical trials.

Meanwhile, with the deepening of research on Defactinib, more compounds based on Defactinib have been developed, which have better effect, or have better metabolic stability and pharmacokinetic performance. Patent CN110452229A has disclosed a deuterated Defactinib compound; patent CN111377871A has disclosed a FAK inhibitor and its drug combination. These patents provide more options for the research of FAK inhibitors.

However, for the available synthesis of aminopyrimidine FAK inhibitor compounds, there are certain problems, such as high cost, low yield, and low purity. For example, for the synthesis of deuterated Defactinib in patent CN110452229A, deuterated methylamine hydrochloride, an expensive reagent, is used in the first step of the disclosed route, that not only leads to high cost, but also introduces deuterium sources too early, resulting in a significant loss of yield after multistep synthesis. The total yield of the following synthesis route is only 11%, and thus it is hard to perform a scale-up preparation.

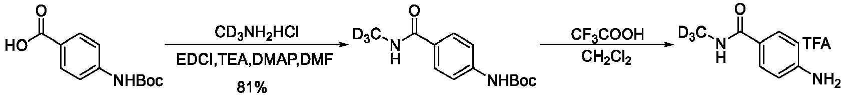

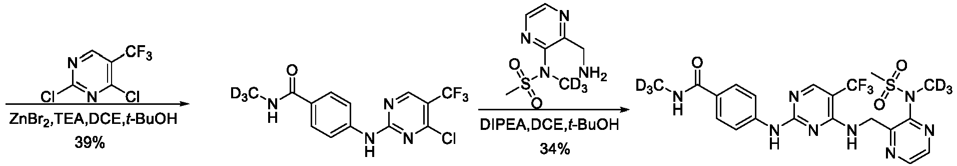

Therefore, there is an urgent need for a simple method for synthesizing aminopyrimidine FAK inhibitor compounds, with high yield and purity.

### Content of the invention

The object of the present invention is to provide an optimized method for synthesizing aminopyrimidine FAK inhibitor compounds.

The present invention provides a method for synthesizing aminopyrimidine FAK inhibitor compounds, which comprises the following steps: 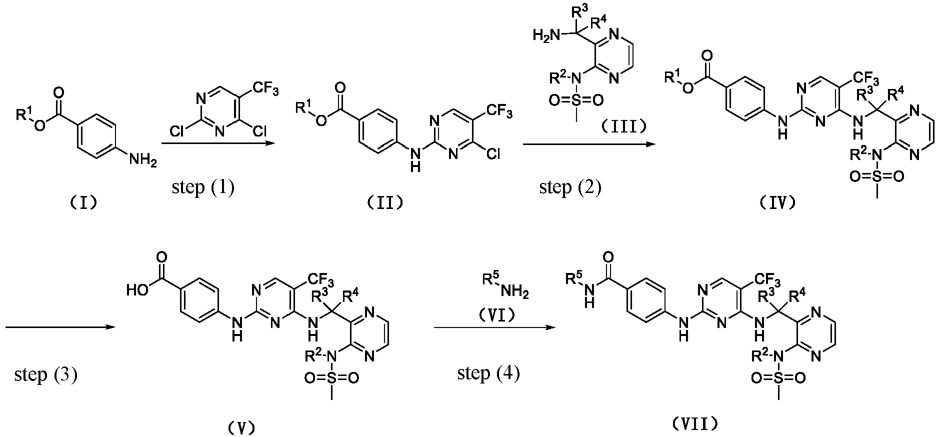
step (1): Compound (**I**), as the starting material, reacts with 2,4-dichloro-5-(trifluoromethyl)pyrimidine in a solvent at the presence of a base, to prepare compound (**II**), wherein R¹ is a hydrogen or a carboxylic acid protecting group;
step (2): In a solvent, compound (**II**) and compound (**III**) react in the presence of a base, to prepare compound (**IV**), wherein R² is selected from the group consisting of C₁-C₆ alkyl or C₁-C₆ deuterated alkyl, and R³ and R⁴ are each independently selected from hydrogen or deuterium; step (3): Carboxylic acid compound (**V**) is prepared from compound (**IV**) by a deprotection reaction;
step (4): Compound (**VII**) is synthesized from compound (**V**) and compound (**VI**) by amide condensation reaction, wherein R⁵ is selected from the group consisting of C₁-C₆ alkyl or C₁-C₆ deuterated alkyl.

Further, in step (1), the molar ratio of compound (**I**), 2,4-dichloro-5-(trifluoromethy)pyrimidine, and the base is (1-3): (1-3): (1-7);
and/or, in step (2), the molar ratio of compound (**II**), compound (**III**), and the base is (1-5): (0.1-1): (1-5);
and/or, in step (4), the molar ratio of compound (**V**) and compound (**VI**) is (1-5):(1-5);
preferably,
in step (1), the molar ratio of compound (**I**), 2,4-dichloro-5-(trifluoromethy)pyrimidine, and the base is 2.0:2.1:6.4;
and/or, in step (2), the molar ratio of compound (**II**), compound (**III**), and the base is 1:0.5:1.5; and/or, in step (4), the molar ratio of compound (**V**) and compound (**VI**) is 1:1.1.

Further, in step (1), the reaction temperature is ranged from -20 °C to 150 °C; and/or, in step (2), the reaction temperature is ranged from -20 °C to 150 °C; and/or, in step (3), the reaction temperature is ranged from -20 °C to 150 °C; and/or, in step (4), the reaction temperature is ranged from -20 °C to 150 °C;
preferably, in step (1), the reaction temperature is ranged from 20 °C to 30 °C; and/or, in step (2), the reaction temperature is ranged from 60 °C to 80 °C; and/or, in step (3), the reaction temperature is ranged from 20 °C to 30 °C; and/or, in step (4), the reaction temperature is ranged from 20 °C to 30 °C.

Further, in step (1), R¹ is selected from the group consisting of hydrogen, ester group, silylester group, thioester group, tin ester group, amide, hydrazine amide, alkyl, alkenyl, alkynyl, unsaturated aliphatic ring, aromatic ring, heterocycle or aromatic heterocycle;
preferably, in step (1), R¹ is selected from the group consisting of hydrogen, ester group, silylester group, thioester group, tin ester group, amide, hydrazine amide, C₁-C₆ alkyl, C₂-C₆ alkenyl, C₂-C₆ alkynyl, unsaturated aliphatic ring, aromatic ring, heterocycle or aromatic heterocycle;
more preferably, in step (1), R¹ is selected from the group consisting of hydrogen, methyl, ethyl, n-propyl, isopropyl, n-butyl, sec-butyl, tert-butyl, amyl, hexyl, N,N-diethylaminoethyl, allyl, propinyl, unsaturated aliphatic ring, aromatic ring, heterocycle or aromatic heterocycle;
further preferably, in step (1), R¹ is selected from the group consisting of hydrogen, methyl, ethyl, and tert-butyl.

Further, in step (1), the base is selected from the group consisting of triethylamine, diethylamine, N,N-diisopropylethylamine, triethylenediamine, 4-dimethylaminopyridine, N,N-dimethylaniline, 1, 8-diazobicyclo [5,4, 0]undec-7-ene, pyridine, N-methylmorpholine, tetramethylethylenediamine, sodium carbonate, potassium carbonate, cesium carbonate, potassium tert-butoxide, sodium tert-butoxide, sodium methoxide or sodium ethoxide; preferably, in step (1), the base is triethylamine.

Further, in step (1), the solvent is a mixed solvent composed of any one or more of dichloromethane, trichloromethane, carbon tetrachloride, dichloroethane, acetonitrile, methanol, ethanol, isopropanol or tert-butanol;
preferably, in step (1), the solvent is a mixed solvent of dichloroethane and tert-butanol;
more preferably, in step (1), the volume ratio of dichloroethane to tert-butanol is 1:1.

Further, in step (2), R² is selected from the group consisting of methyl or deuteromethyl; and/or, in step (2), the base is selected from the group consisting of triethylamine, diethylamine, N,N-diisopropylethylamine, triethylenediamine, 4-dimethylaminopyridine, N,N-dimethylaniline, 1,8-diazobicyclo[5,4,0]undec-7-ene, pyridine, N-methylmorpholine, tetramethylethylenediamine, sodium carbonate, potassium carbonate, cesium carbonate, potassium tert-butoxide, sodium tert-butoxide, sodium methoxide or sodium ethoxide;
preferably, in step (2), the base is N,N-diisopropylethylamine.

Further, in step (2), the solvent is a mixed solvent composed of any one or more of methanol, ethanol, isopropanol, acetonitrile, N,N-dimethylformamide, N,N-dimethylacetamide, N-methylpyrrolidone, or dimethylsulfoxide;
preferably, in step (2), the solvent is N-methylpyrrolidone.

Further, in step (3), the solvent used in the deprotection reaction is a mixed solvent composed of any one or more of water, tetrahydrofuran, dioxane, methanol, ethanol, isopropanol, or dichloromethane;
preferably, in step (3), the solvent used in the deprotection reaction is dioxane.

Further, in step (3), the deprotection reagents used in the deprotection reaction are acids or bases; preferably, in step (3), the deprotection reagents used in the deprotection reaction are selected from the group consisting of hydrochloric acid, trifluoroacetic acid, lithium hydroxide, sodium hydroxide or potassium hydroxide;
more preferably, in step (3), provided that R¹ is tert-butyl, the deprotection reagent used in the deprotection reaction is hydrochloric acid.

Further, in step (4), R⁵ is selected from methyl or deuteromethyl; and/or, in step (4), the amide condensation reaction is carried out in the presence of a condensation agent, and the condensation agent is selected from the group consisting of isopropyl chloroformate, N,N'-carbonyldiimidazole, p-toluenesulfonyl chloride, (Boc)₂O, dicyclohexylcarbodiimide, diisopropylcarbodiimide, 1-(3-dimethylaminopropyl)-3-ethylcarbodiimide, *O*-(7-azabenzotriazol-1-yl)-bis(dimethylamino)carbenium hexafluorophosphate, *O*-(benzotriazol-1-yl)-bis(dimethylamino)carbenium hexafluorophosphate, *O*-(5-chlorobenzotriazol-1-yl)-bis(dimethylamino)carbenium hexafluorophosphate, *O*-(benzotriazol-1-yl)-bis(dimethylamino)carbenium tetrafluoroborate, *O*-(N-succinimide)-bis (dimethylamino)carbenium tetrafluoroborate, *O*-(*N*-endo-5-norcamphene-2,3-dicarbodiimide)-bis(dimethylamino)carbenium tetrafluoroborate, benzotriazol-1-yloxy-tri(dimethylamino) phosphonium hexafluorophosphate, benzotriazol-1-yloxy-tris(tetrahydropyrrolyl)phosphonium hexafluorophosphate, diphenylphosphoryl chloride, diethyl cyanophosphate, diphenylphosphoryl azide, thiodimethylphosphoryl azide, or bis(2-oxo-3-oxazolidinyl)phosphoryl chloride;
preferably, in step (4), the condensation agent is N,N'-carbonyldiimidazole;
more preferably, the molar ratio of compound (V) to the condensation agent is (1-5):(1-5); further preferably, the molar ratio of compound (V) to the condensation agent is 1:1.1.

Further, in step (4), the solvent used for the amide condensation reaction is an aprotic solvent; preferably, in step (4), the solvents used for the amide condensation reaction are selected from the group consisting of dichloromethane, dichloroethane, acetone, diethyl ether, diisopropyl ether, tetrahydrofuran, dioxane, toluene, acetonitrile, N,N-dimethylformamide, N,N-dimethylacetamide, or dimethyl sulfoxide;
more preferably, in step (4), the solvent used for the amide condensation reaction is dichloromethane.

Further, compound (**III**) was prepared by the following procedures: 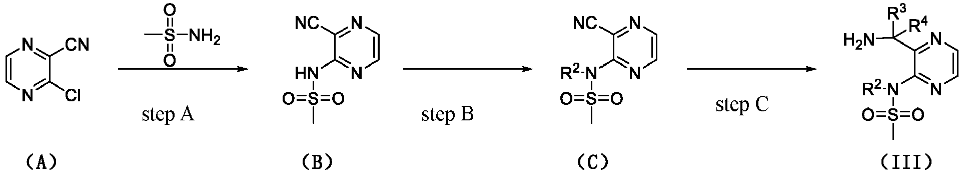
step A: In a solvent, compound (**B**) is synthesized by a substitution reaction of compound (A), as the starting material, with methanesulfonamide in the presence of a base;
step B: In a solvent, compound (**B**) reacts with a methylation reagent in the presence of a base, to obtain compound (**C**), wherein R² is selected from the group consisting of C₁-C₆ alkyl or C₁-C₆ deuterated alkyl
step C: compound (**III**) is prepared by reaction of compound (**C**) with a reducing reagent, wherein R³ and R⁴ are each independently selected from hydrogen or deuterium.

Further, in step A, the molar ratio of compound (**A**), methanesulfonamide, and the base is (1-5): (1-5): (1-5);
and/or, in step B, the molar ratio of compound (**B**), the base, and the methylation reagent is (1-2): (1-5): (1-3);
preferably,
in step A, the molar ratio of compound (**A**), methane sulfonamide, and the base is 2:3:4;
and/or, in step B, the molar ratio of compound (**B**), the base, and the methylation reagent is 1.5:4.5:2.3.

Further, in step A, the reaction temperature is ranged from -20 °C to 150 °C; and/or, in step B, the reaction temperature is ranged from -20 °C to 150 °C; and/or, in step C, the reaction temperature is ranged from -20 °C to 150 °C;
preferably, in step A, the reaction temperature is ranged from 80 °C to 100 °C; and/or, in step B, the reaction temperature is ranged from 60 °C to 80 °C; and/or, in step C, the reaction temperature is ranged from 20 °C to 30 °C.

Further, in step A, the base is selected from the group consisting of sodium carbonate, sodium bicarbonate, potassium carbonate, potassium bicarbonate, cesium carbonate, sodium hydroxide, potassium hydroxide, lithium hydroxide, calcium hydroxide, potassium tert-butoxide, sodium tert-butoxide, sodium methoxide, sodium ethoxide, triethylamine, diethylamine, N,N-diisopropylethylamine, triethylenediamine, 4-dimethylaminopyridine, N,N-dimethylaniline, 1,8-diazobicyclo[5,4,0]undec-7-ene, pyridine, N-methylmorpholine, or tetramethylethylenediamine; preferably, the base is cesium carbonate.

Further, in step A, the solvent is selected from the group consisting of methanol, ethanol, isopropanol, ethyl acetate, dichloromethane, acetonitrile, N,N-dimethylformamide, N,N-dimethylacetamide, dimethyl sulfoxide, N-methylpyrrolidone, tetrahydrofuran, toluene, dichloromethane, dioxane or water;
preferably, in step A, the solvent is acetonitrile.

Further, in step B, R² is selected from methyl or deuteromethyl; and/or, in step B, the base used is selected from the group consisting of sodium carbonate, sodium bicarbonate, potassium carbonate, potassium bicarbonate, cesium carbonate, sodium hydroxide, potassium hydroxide, lithium hydroxide, calcium hydroxide, potassium tert-butoxide, sodium tert-butoxide, sodium methoxide, sodium ethoxide, triethylamine, diethylamine, N,N-diisopropylethylamine, triethylenediamine, 4-dimethylaminopyridine, N,N-dimethylaniline, 1,8-diazobicyclo[5,4,0]undec-7-ene, pyridine, N-methylmorpholine, or tetramethylethylenediamine; preferably, the base is potassium carbonate.

Further, in step B, the methylation reagents are selected from the group consisting of non-deuterated or deuterated methanol, iodomethane, dimethyl sulfate, methyl p-methylbenzenesulfonate, methyl p-nitrobenzenesulfonate, methyl trifluoromethanesulfonate, dimethyl carbonate, trimethyl phosphite, dimethyl phosphite, trimethyl phosphate, dimethyl phosphite, trimethyl orthoformate, trimethyl orthoacetate, N-methyl methanesulfonamide, and diazomethane;
preferably, in step B, the methylation reagents are iodomethane and deuterated iodomethane. Further, in step B, the solvent used is selected from the group consisting of methanol, ethanol, isopropanol, ethyl acetate, dichloromethane, acetonitrile, N,N-dimethylformamide, N,N-dimethylacetamide, dimethyl sulfoxide, N-methylpyrrolidone, tetrahydrofuran, toluene, dichloromethane, dioxane or water;
preferably, the solvent used is acetonitrile or N,N-dimethylformamide.

Further, in step C, the reducing reagents used are selected from the group consisting of lithium aluminum hydride, diisobutyl aluminum hydride, sodium borohydride, lithium borohydride, zinc borohydride, sodium triacetoxyborohydride, sodium cyanoborohydride, lithium triethylborohydride, lithium triisobutylborohydride, lithium N,N-(dimethylamino)borohydride, dimethoxyethoxyaluminum hydride, bis(cyclopentadienyl)dicarbonyl titanium, borane, dimethyl sulfide borane, triethylsilane, zinc-acetic acid, hydrogen or deuterium;
preferably, in step C, the reducing reagent used is hydrogen or deuterium.

Further, in step C, a catalyst is used in the reaction, which is a transition metal catalyst; preferably, in step C, the catalyst is Raney Ni, Pd/C, or Pt/C;
more preferably, in step C, the catalyst is Raney Ni.

Further, in step C, the solvent used in the reaction is a mixed solvent composed of any one or more of methanol, ethanol, isopropanol, tert-butanol, diethyl ether, tetrahydrofuran, toluene, dichloromethane, dioxane or water;
preferably, in step C, the solvent used in the reaction is methanol.

In the present invention, room temperature means 25 ± 5 °C; overnight denotes 12 ± 2 h.

In the present invention, an unsaturated aliphatic ring refers to a cyclic compound which does not contain heteroatoms, but comprises one or more double or triple bonds; aromatic ring refers to a compound with an aromatic ring structure; heterocyclic compounds mean a cyclic compound containing one or more heteroatoms; aromatic heterocycles refer to compounds having one or more heteroatoms and aromatic ring structures.
The present invention provides a method for synthesizing aminopyrimidine FAK inhibitor compounds, which is simple to operate, and has low costs. The product prepared can obtain a high total yield (the total yield of the shortest linear route starting from commercially available raw materials is ≥ 66%) and a high purity (≥ 99%), and the product yield and purity are superior to those in the prior art (in the prior art, the total yield is about 11%, and the purity is 99%). The synthesis method according to the present invention achieves excellent effects, can also be used to successfully prepare a final product on kilogram scale, and is suitable for industrial production, having a good application prospect.

Obviously, based on the above content of the present invention, according to the common technical knowledge and the conventional means in the field, without department from the above basic technical spirits, other various modifications, alternations, or changes can further be made. By the following specific examples of said embodiments, the above content of the present invention is further illustrated. But it should not be construed that the scope of the above subject matter of the present invention is limited to the following examples. The techniques realized based on the above content of the present invention are all within the scope of the present invention.

### Examples

The raw materials and equipment used in the specific examples of the present invention were known products obtained by purchasing commercially available products. In the present invention, unless otherwise stated, (i) the temperature was expressed in degrees Celsius (°C), and the operation was carried out at room temperature or ambient temperature; (ii) the organic solvent was dried over anhydrous sodium sulfate, and the solvent was evaporated under reduced pressure in a rotary evaporator, and the bath temperature was not higher than 60 °C; (iii) the reaction process was detected by thin layer chromatography (TLC) or LCMS.

### Example 1. Synthesis of N-(methyl)-4-((4-((3-(N-(methyl)methanesulfonamido)pyrazin-2-yl)methyl)amino)-5-(trifluoromethyl)pyrimidin-2-yl)amino)benzamide (defactinib)

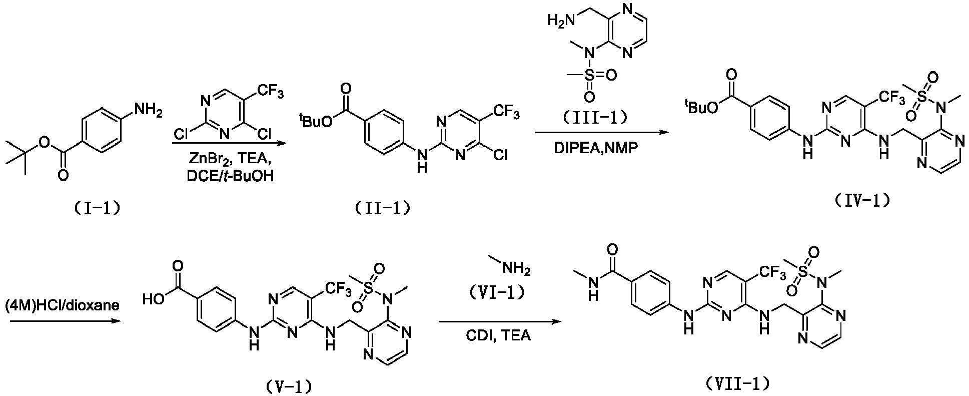

### Step A: Preparation of N-(3-cyanopyrazin-2-yl)methanesulfonamide (intermediate B)

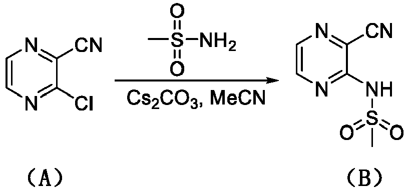

To 100 mL of acetonitrile, were successively added the starting material 2-chloro-3-cyanopyrazine (starting material A) (4.19 g, 30 mmol), methanesulfonamide (4.28 g, 45 mmol) and cesium carbonate (19.5 g, 60 mmol), and then under nitrogen protection, the reaction was heated to 80 °C and allowed to react for 4 h under stirring. The reaction progress was monitored with TLC (PE/EA = 2/1, v/v). After the raw material had completely disappeared, the reaction was cooled to room temperature, and evaporated under reduced pressure to remove acetonitrile. A mixed solvent of dichloromethane/methanol (5/1, v/v) was added for triturating, and then the reaction solution was filtered, and the filter cake was rinsed. The filtrate was dried and concentrated, to obtain N-(3-cyanopyrazin-2-yl)methanesulfonamide (intermediate B, 5.9 g), with a yield of 95%. LC/MS (ESI⁺) calcd for C₆H₆N₄O₂S ( [M+H]⁺) *m*/*z:* 198.0; found 199.1.

### Step B: Preparation of N-(3-cyanopyrazin-2-yl)-N-(methyl)methanesulfonamide (intermediate C-1)

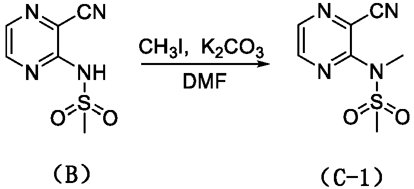

N-(3-cyanopyrazin-2-yl)methanesulfonamide (intermediate B) (2.98 g, 15 mmol), potassium carbonate (6.3 g, 45 mmol), and iodomethane (3.3 g, 23 mmol) were successively added to 30 mL of DMF, and then under nitrogen protection, the reaction solution was heated to 80 °C and allowed to react overnight under stirring. The reaction progress was monitored with TLC (PE/EA = 2/1, v/v). After the raw material had completely disappeared, the reaction was cooled to room temperature, and extracted with 100 mL of ethyl acetate. The organic layer was washed with saturated sodium chloride solution three times (100 mL × 3), and then the organic phase was dried and concentrated to obtain N-(3-cyanopyrazin-2-yl)-N-(methyl)methanesulfonamide (intermediate C-1, 2.6 g), with a yield of 80%. LC/MS (ESI⁺) *m*/*z:* 213 ([M+H]⁺).

### Step C: Preparation of N-(3-(aminomethyl)pyrazin-2-yl)-N-(methyl)methanesulfonamide (intermediate III-1)

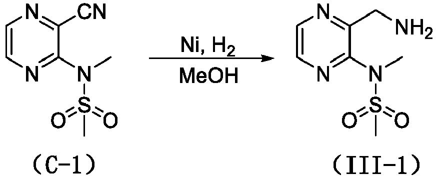

N-(3-cyanopyrazin-2-yl)-N-(methyl)methanesulfonamide (intermediate **C-1**) (1.1 g, 5 mmol) was dissolved in 20 mL of methanol, to which was added about 1 g of Raney Ni catalyst, and then the reaction system was replaced with hydrogen three times. The reaction solution was stirred at room temperature overnight, and the reaction progress was detected by TLC (DCM/MeOH = 5/1, v/v). After the raw material disappeared, the reaction solution was filtered through diatomaceous earth, and the filtrate was concentrated to obtain N-(3-(aminomethyl)pyrazin-2-yl)-N-(methyl)methanesulfonamide (intermediate **III-1**, 0.88 g), with a yield of 83%. LC/MS (ESI+) *m*/*z:* 217 ([M+H]⁺).

### Step 1: Preparation of tert-butyl 4-((4-chloro-5-(trifluoromethyl)pyrimidin-2-yl)amino)benzoate (intermediate II-1)

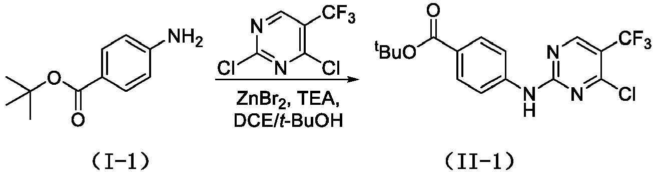

The raw material 2,4-dichloro-5-trifluoromethylpyrimidine (4.56 g, 21 mmol) was dissolved in 100 mL mixed solvent of dichloroethane/tert-butanol (1/1, v/v), and then cooled in an ice water bath under nitrogen protection, to which was added zinc bromide (13.5 g, 60 mmol). The reaction solution was further stirred for 30 min, and then tert-butyl 4-aminobenzoate (raw material **I-1**) (3.86 g, 20 mmol) and triethylamine (6.46 g, 64 mmol) were sequentially added. The ice water bath was removed, and then the reaction solution was allowed to naturally warmed to room temperature and react overnight. TLC (PE/EA = 5/1, v/v) was used to monitor the reaction progress. After completion of the reaction, the reaction solution was directly filtered, rinsed with dichloroethane, and the filter cake was dried to obtain tert-butyl 4-((4-chloro-5-(trifluoromethyl)pyrimidin-2-yl)amino)benzoate (intermediate **II-1**, 7.35 g), as a white solid, with a yield of 97% and a purity of 94.68%. LC/MS (ESI+) *m*/*z:* 373 ([M+H]⁺).

### Step 2: Preparation of tert-butyl 4-((4-((3-(N-(methyl)methanesulfonamido)pyrazin-2-yl)methyl)amino)-5-(trifluoromethyl)pyrimidin-2-yl)amino)benzoate (intermediate IV-1)

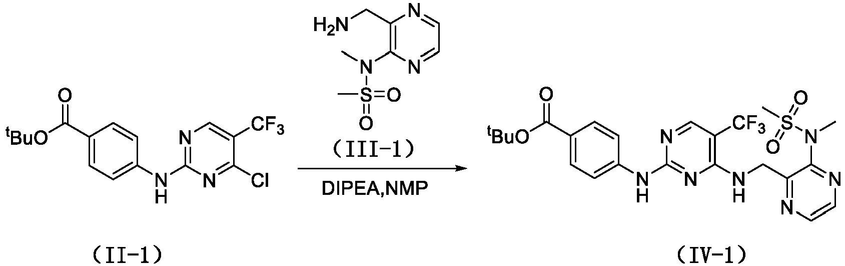

tert-Butyl 4-((4-chloro-5-(trifluoromethyl)pyrimidin-2-yl)amino)benzoate (intermediate II-1) (374 mg, 1.0 mmol), N-(3-(aminomethyl)pyrazin-2-yl)-N-(methyl)methanesulfonamide (intermediate **III-1**) (110 mg, 0.5 mmol), and N,N-diisopropylethylamine (195 mg, 1.5 mmol) were successively added to 25 mL of N-methylpyrrolidone, and then under nitrogen protection, the reaction solution was heated to 60 °C, and allowed to react for 6 h under stirring. The reaction progress was monitored by TLC (PE/EA = 5/1, v/v) and LCMS. After disappearance of intermediate **III-1**, the reaction solution was cooled to room temperature, extracted with 50 mL of ethyl acetate, and then washed three times with saturated sodium chloride solution (40 mL × 3). The organic phase was dried, concentrated, and purified by pre-TLC (PE/EA = 2/1, v/v), to provide tert-butyl 4-((4-((3-(N-(methyl)methanesulfonamido)pyrazin-2-yl)methyl)amino)-5-(trifluoromethyl)pyrimidin-2-yl)amino)benzoate (intermediate IV-1, 210 mg), with a yield of 76% (based on the intermediate III-1) and a purity of 92.24%. LC/MS (ESI+) *m*/*z:* 554 ([M+H]⁺).

### Step 3: Preparation of 4-((4-((3-(N-(methyl)methanesulfonamido)pyrazin-2-yl)methyl)amino)-5-(trifluoromethyl)pyrimidin-2-yl)amino)benzoic acid (intermediate V-1)

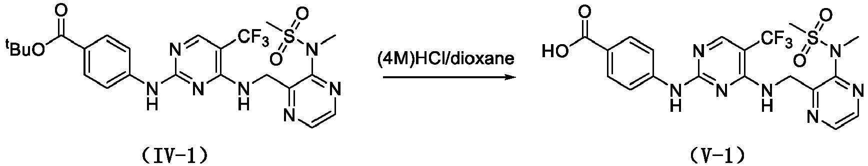

tert-Butyl 4-((4-((3-(N-(methyl)methanesulfonamido)pyrazin-2-yl)methyl)amino)-5-(trifluoromethyl)pyrimidin-2-yl)amino)benzoate (intermediate IV-1) (210 mg, 0.38 mmol) was dissolved in 10 mL of HCl/dioxane (the concentration of HCl was 4 M), and then reacted at room temperature for 3 h under stirring. Lots of white precipitates were observed. The reaction progress was detected by TLC (PE/EA= 5/1, v/v) and LCMS. After completion of the reaction, the reaction solution was directly concentrated under reduced pressure, to remove dioxane. Petroleum ether was added for triturating, and then the resultant solution was filtered and rinsed, to obtain 4-((4-((3-(N-(methyl-d3)methanesulfonamido)pyrazin-2-yl)methyl)amino)-5-(trifluoromethyl) pyrimidin-2-yl)amino)benzoic acid (intermediate **V-1**, 195 mg), as a white solid, with a yield of 93% and a purity of 93.7%. LC/MS (ESI+) *m*/*z:* 498 ([M+H]⁺).

### Step 4: Preparation of N-(methyl)-4-((4-((3-(N-(methyl)methanesulfonamido)pyrazin-2-yl)methyl)amino)-5-(trifluoromethyl)pyrimidin-2-yl)amino)benzamide (target compound VII-1)

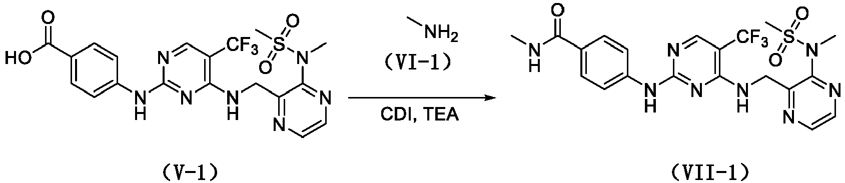

4-((4-((3-(N-(methyl)methanesulfonamido)pyrazin-2-yl)methyl)amino)-5-(trifluoromethyl) pyrimidin-2-yl)amino)benzoic acid (intermediate **V-1**) (115 mg, 0.20 mmol) and N,N'-carbonyldiimidazole (36 mg, 0.22 mmol) were dissolved in 20 mL of dichloromethane, and then stirred under nitrogen protection at room temperature for about 3 h, until the solid was dissolved and the reaction solution became clear. After disappearance of the raw material was detected with TLC (DCM/MeOH = 10/1, v/v), triethylamine (202 mg, 2.0 mmol) and methylamine hydrochloride (raw material **VI-1**) (16 mg, 0.22 mmol) were added in sequence, and the resultant solution was stirred and reacted at room temperature for 2 h. After completion of the reaction monitored using TLC (DCM/MeOH = 10/1, v/v), the reaction solution was extracted with dichloromethane, and then successively washed with saturated ammonium chloride solution and saturated sodium chloride solution. The organic phase was dried and rotatory evaporated, and the residue was purified by prep-TLC (DCM/MeOH = 10/1, v/v), to afford N-(methyl)-4-((4-((3-(N-(methyl)methanesulfonamido)pyrazin-2-yl)methyl)amino)-5-(trifluoromethyl)pyrimidin-2-yl)amino)benzamide (target compound **VII-1**, 94 mg), with a yield of 94% and a purity of 100%. LC/MS (ESI+) *m*/*z:* 511 ([M+H]⁺). ¹H NMR (400 MHz, DMSO-*d*₆) *δ* 9.83 (s, 1H), 8.69 (d, *J* = 2.5 Hz, 1H), 8.58 (d, *J* = 2.3 Hz, 1H), 8.31 (s,1H), 8.20 (d, *J* = 4.5 Hz, 1H), 7.63 (q, *J* = 8.7 Hz, 4H), 7.41 (t, *J* = 4.9 Hz, 1H), 5.00 (d, *J* = 4.9 Hz, 2H), 3.21 (d, *J* = 10.5 Hz, 6H), 2.75 (d, *J* = 4.4 Hz, 3H).

### Example 2. Synthesis of N-(methyl-d3)-4-((4-((3-(N-(methyl-d3)methanesulfonamido) pyrazin-2-yl)methyl)amino)-5-(trifluoromethyl)pyrimidin-2-yl)amino)benzamide (VII-2)

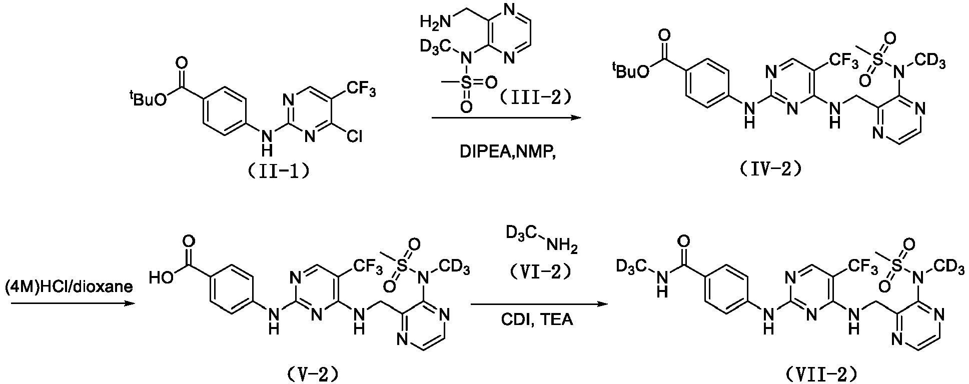

### Step B: Preparation of N-(3-cyanopyrazin-2-yl)-N-(methyl-d3)methanesulfonamide (intermediate C-2)

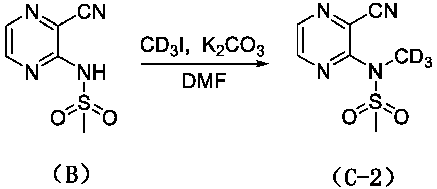

Using intermediate compound **B** and deuterated iodomethane as raw materials, intermediate **C-2** was prepared by using the reaction conditions similar to step B of Example 1, with a yield of 80%. LC/MS (ESI+) *m*/*z:* 216 ([M+H]⁺).

### Step C: Preparation of N-(3-(aminomethyl)pyrazin-2-yl)-N-(methyl-d₃) methanesulfonamide (intermediate III-2)

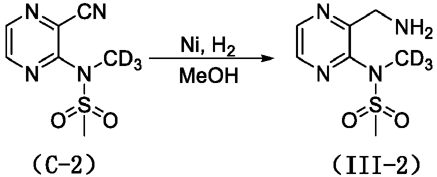

Using intermediate **C-2** as raw material, intermediate **III-2** was prepared by using the reaction conditions similar to step C of Example 1, with a yield of 83%. LC/MS (ESI+) *m*/*z:* 220 ([M+H]⁺).

### Step 2: Preparation of tert-butyl 4-((4-((3-(N-(methyl-d3)methanesulfonamido)pyrazin-2-yl)methyl)amino)-5-(trifluoromethyl)pyrimidin-2-yl)amino)benzoate (intermediate IV-2)

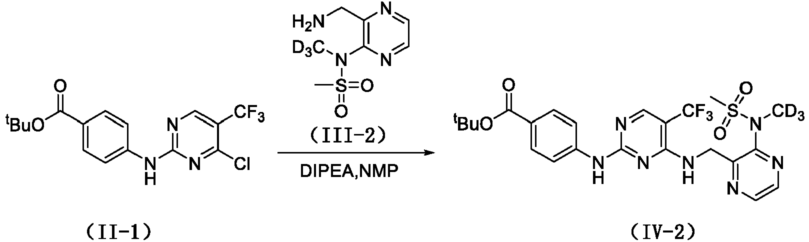

Using intermediate **II-1** and intermediate III-2 as raw materials, intermediate IV-2 was prepared by using the reaction conditions similar to step 2 of Example 1, with a yield of 76%. LC/MS (ESI+) *m*/*z:* 557 ([M+H]⁺).

### Step 3: Preparation of 4-((4-((3-(N-(methyl-d3)methanesulfonamido)pyrazin-2-yl)methyl)amino)-5-(trifluoromethyl)pyrimidin-2-yl)amino)benzoic acid (intermediate V-2)

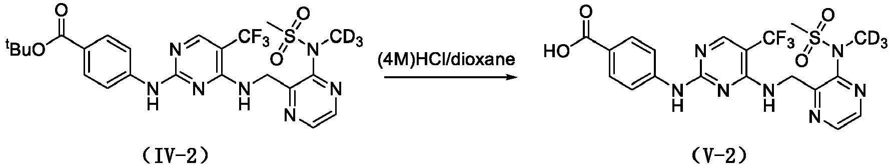

Using intermediate (**IV-2**) as raw material, intermediate **V-2** was prepared by using the reaction conditions similar to step 3 of Example 1, with a yield of 93%. LC/MS (ESI+) m/z: 501 ([M+H]⁺).

### Step 4: Preparation of N-(methyl-d3)-4-((4-((3-(N-(methyl-d3)methanesulfonamido) pyrazin-2-yl)methyl)amino)-5-(trifluoromethyl)pyrimidin-2-yl)amino)benzamide (VII-2)

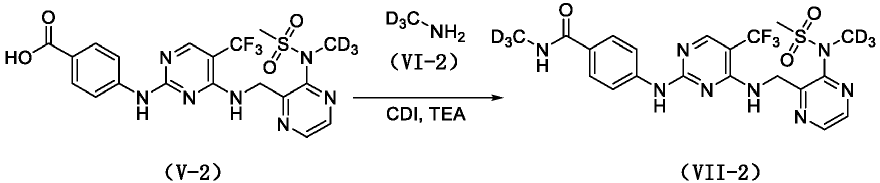

Using intermediate **V-2** and deuterated methylamine hydrochloride **VI-2** as raw materials, intermediate **VII-2** was prepared by using the reaction conditions similar to step 4 of Example 1, with a yield of 94% and a purity of 100%. LC/MS (ESI+) *m*/*z:* 517 ([M+H]⁺). ¹H NMR (400 MHz, DMSO-*d*₆) *δ* 9.83 (s, 1H), 8.69 (d, *J*= 2.8 Hz, 1H), 8.58 (d, *J* = 2.4Hz, 1H), 8.31 (s, 1H), 8.17 (s, 1H), 7.67-7.60 (dd, *J* = 15.4, 8.6 Hz, 4H), 7.42-7.39 (t, *J* = 5.2Hz, 1H), 5.00 (d, *J* = 4.8 Hz, 2H), 3.20 (s, 3H).

### Exmaple 3: Synthesis of N-(methyl-d3)-4-((4-((3-(N-(methyl)methanesulfonamido)pyrazin-2-yl)methyl)amino)-5-(trifluoromethyl)pyrimidin-2-yl)amino)benzamide (VII-3)

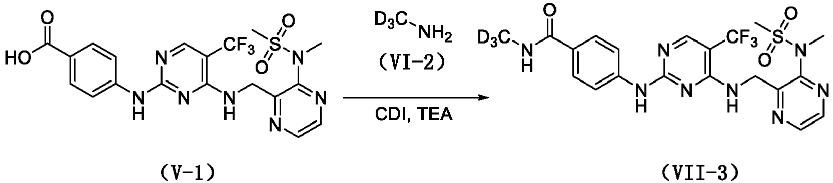

Using intermediate **V-1** and deuterated methylamine hydrochloride (**VI-2**) as raw materials, intermediate **VII-3** was prepared by using the reaction conditions similar to step 4 of Example 1, with a yield of 94% and a purity of >97.57%. LC/MS (ESI+) *m*/*z:* 514 ( [M+H]⁺). ¹H NMR (400 MHz, DMSO-*d*₆) *δ* 9.86 (s, 1H), 8.69 (d, *J*= 2.4 Hz, 1H), 8.58 (d, *J* = 2.8Hz, 1H), 8.32 (s, 1H), 8.18 (s, 1H), 7.67-7.59 (dd, *J* = 19.6, 8.8 Hz, 4H), 7.48-7.45 (t, *J* = 5.2Hz, 1H), 5.00 (d, *J* = 4.8 Hz, 2H), 3.22 (s, 3H), 3.20 (s, 3H).

### Example 4. Kilogram-scale synthesis of N-(methyl-d3)-4-((4-((3-(N-(methyl-d3)methanesulfonamido)pyrazin-2-yl)methyl)amino)-5-(trifluoromethyl)pyrimidin-2-yl)amino)benzamide (VII-2)

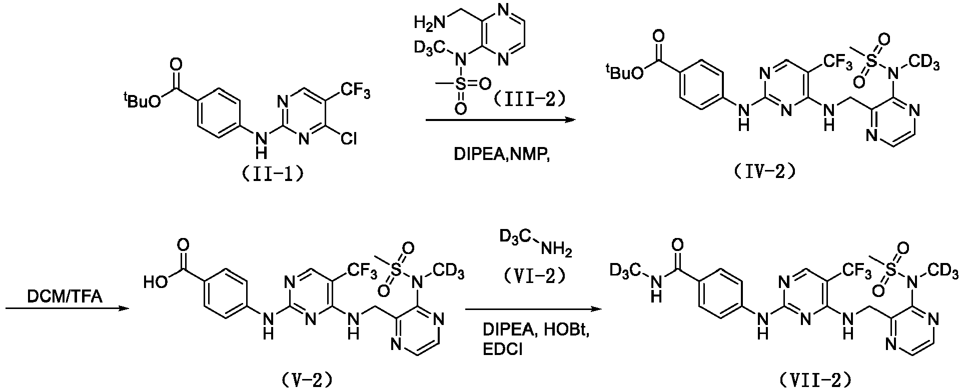

### Step A: Preparation of tert-butyl 4-((4-((3-(N-(methyl)methanesulfonamido)pyrazin-2-yl)methyl)amino)-5-(trifluoromethyl)pyrimidin-2-yl)amino)benzoate (intermediate IV-2) tert-Butyl 4-((4-chloro-5-(trifluoromethyl)pyrimidin-2-yl)amino)benzoate (intermediate II-1)

(4.0 kg, 10.7 mol), N-(3-(amino-methyl-d3)pyrazin-2-yl)-N-(methyl)methanesulfonamide (intermediate **III-2**) (3.56 kg, 13.91 mol), and N,N-diisopropylethylamine (4.15 kg, 32.11 mol) were sequentially added to 40 kg of N-methylpyrrolidone, and then under nitrogen protection, the reaction solution was heated to 60 °C, and allowed to react for 2-3 h under stirring. The reaction progress was monitored by TLC (PE/EA = 5/1, v/v) and LCMS. After disappearance of intermediate **III-2**, the reaction solution was cooled to room temperature, and then slowly added to purified water (57 kg), resulting in a large amount of solid precipitation. The resultant solution was stirred for 1-2 h, and then the solid was collected by vacuum filtration, to obtain tert-butyl 4-((4-((3-(N-(methyl-d₃)methanesulfonamido)pyrazin-2-yl)methyl-d₃)amino)-5-(trifluoromethyl) pyrimidin-2-yl)amino)benzoate (intermediate **IV-2**, 5.9 kg), with a yield of 99%. LC/MS (ESI+) *m*/*z:* 557 ([M+H]⁺).

### Step B: Preparation of 4-((4-((3-(N-(methyl-d3)methanesulfonamido)pyrazin-2-yl)methyl)amino)-5-(trifluoromethyl)pyrimidin-2-yl)amino)benzoic acid (intermediate V-2)

5.8 kg of tert-butyl 4-((4-((3-(N-(methyl-d3)methanesulfonamido)pyrazin-2-yl)methyl-d3)amino)-5-(trifluoromethyl)pyrimidin-2-yl)amino)benzoate (intermediate **IV-2**) was added to 58 L of dichloromethane, to which was then added 14.26 kg of trifluoroacetic acid, and the resultant solution was stirred at 30-40 °C for 2-3 h. The reaction sample was collected to monitor the reaction progress, and after completion of the reaction, the reaction solution was concentrated to dry. Then, 29 L of ethyl acetate was added, and the reaction solution was stirred for 1-2 h, filtered, and dried, to provide 4-((4-((3-(N-(methyl-d₃)methanesulfonamido)pyrazin-2-yl)methyl)amino)-5-(trifluoromethyl)pyrimidin-2-yl)amino)benzoic acid (6.0 kg), with a yield of 100%. LC/MS (ESI+) *m*/*z:* 501 ([M+H]⁺).

### Step C: Preparation of N-(methyl-d3)-4-((4-((3-(N-(methyl-d3)methanesulfonamido) pyrazin-2-yl)methyl)amino)-5-(trifluoromethyl)pyrimidin-2-yl)amino)benzamide (VII-2)

5.9 kg of 4-((4-((3-(N-(methyl-d₃)methanesulfonamido)pyrazin-2-yl)methyl)amino)-5-(trifluoromethyl)pyrimidin-2-yl)amino)benzoic acid (intermediate V-2) was added to 59 L of NMP, and then the mixture was stirred to dissolve, to which was added 6.9 kg of DIPEA. The resultant solution was stirred for 15 min, and then, 3.98 kg of HOBt, 5.65 kg of EDCI, and 0.99 kg of deuterated methylamine hydrochloride were added in sequence, and allowed to react for 2-3 h. The reaction sample was collected to detect the reaction progress, and after completion of the reaction, purified water (30 V) was slowly added to the reaction solution. The temperature of the reaction solution was kept at 20-30 °C, and a large amount of solid precipitated. After stirring at 20-30 °C for 0.5-2 h, the solid was collected by filtering and then dried, to obtain N-(methyl-d₃)-4-((4-((3-(N-(methyl-d3)methanesulfonamido)pyrazin-2-yl)methyl)amino)-5-(trifluoromethyl)pyrimidin-2-yl)amino)benzamide (4.18 kg), with a yield of 69% and a purity of 99.25%. LC/MS (ESI+) *m*/*z:* 517 ([M+H]⁺).

In summary, the present invention provided a method for synthesizing aminopyrimidine FAK inhibitor compounds, which was easy to operate and low in cost; the prepared product could achieve a high total yield (≥66%) and purity (≥99%), that were superior to the prior art (with a total yield of about 11% and a purity of 99%). The synthesis method of the present invention had achieved excellent results, and could also be used to successfully prepare kilogram-scale products, which were suitable for industrial production and had good application prospects.

## Claims

1. A method for synthesizing aminopyrimidine FAK inhibitor compounds, **characterized in that** it comprises the following steps: 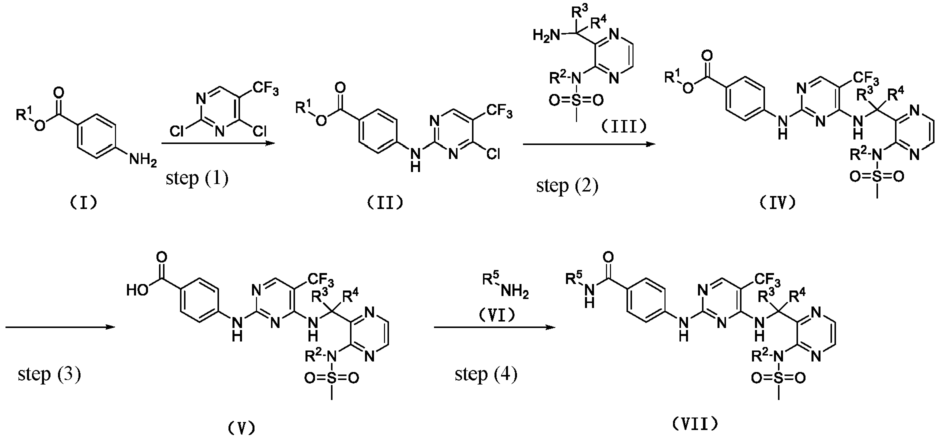
step (1): Compound (I), as the starting material, reacts with 2,4-dichloro-5-(trifluoromethyl)pyrimidine in a solvent at the presence of a base, to prepare compound (II), wherein R¹ is a hydrogen or a carboxylic acid protecting group;
step (2): In a solvent, compound (II) and compound (III) react in the presence of a base, to prepare compound (IV), wherein R² is selected from the group consisting of C₁-C₆ alkyl or C₁-C₆ deuterated alkyl, and R³ and R⁴ are each independently selected from hydrogen or deuterium; step (3): Carboxylic acid compound (**V**) is prepared from compound (**IV**) by a deprotection reaction;
step (4): Compound (**VII**) is synthesized from compound (**V**) and compound (**VI**) by amide condensation reaction, wherein R⁵ is selected from the group consisting of C₁-C₆ alkyl or C₁-C₆ deuterated alkyl.

2. The method according to claim 1, **characterized in that** in step (1), the molar ratio of compound (**I**), 2,4-dichloro-5-(trifluoromethy)pyrimidine, and the base is (1-3): (1-3): (1-7);
and/or, in step (2), the molar ratio of compound (**II**), compound (**III**), and the base is (1-5): (0.1-1): (1-5);
and/or, in step (4), the molar ratio of compound (**V**) and compound (**VI**) is (1-5):(1-5); preferably,
in step (1), the molar ratio of compound (**I**), 2,4-dichloro-5-(trifluoromethy)pyrimidine, and the base is 2.0:2.1:6.4;
and/or, in step (2), the molar ratio of compound (**II**), compound (**III**), and the base is 1:0.5:1.5;
and/or, in step (4), the molar ratio of compound (**V**) and compound (**VI**) is 1:1.1.

3. The method according to claim 1, **characterized in that** in step (1), the reaction temperature is ranged from -20 °C to 150 °C; and/or, in step (2), the reaction temperature is ranged from -20 °C to 150 °C; and/or, in step (3), the reaction temperature is ranged from -20 °C to 150 °C; and/or, in step (4), the reaction temperature is ranged from -20 °C to 150 °C;
preferably, in step (1), the reaction temperature is ranged from 20 °C to 30 °C; and/or, in step (2), the reaction temperature is ranged from 60 °C to 80 °C; and/or, in step (3), the reaction temperature is ranged from 20 °C to 30 °C; and/or, in step (4), the reaction temperature is ranged from 20 °C to 30 °C.

4. The method according to claim 1, **characterized in that** in step (1), R¹ is selected from the group consisting of hydrogen, ester group, silylester group, thioester group, tin ester group, amide, hydrazine amide, alkyl, alkenyl, alkynyl, unsaturated aliphatic ring, aromatic ring, heterocycle or aromatic heterocycle;
preferably, in step (1), R¹ is selected from the group consisting of hydrogen, ester group, silylester group, thioester group, tin ester group, amide, hydrazine amide, C₁-C₆ alkyl, C₂-C₆ alkenyl, C₂-C₆ alkynyl, unsaturated aliphatic ring, aromatic ring, heterocycle or aromatic heterocycle; more preferably, in step (1), R¹ is selected from the group consisting of hydrogen, methyl, ethyl, n-propyl, isopropyl, n-butyl, sec-butyl, tert-butyl, amyl, hexyl, N,N-diethylaminoethyl, allyl, propinyl, unsaturated aliphatic ring, aromatic ring, heterocycle or aromatic heterocycle;
further preferably, in step (1), R¹ is selected from the group consisting of hydrogen, methyl, ethyl, and tert-butyl.

5. The method according to claim 1, **characterized in that** in step (1), the base is selected from the group consisting of triethylamine, diethylamine, N,N-diisopropylethylamine, triethylenediamine, 4-dimethylaminopyridine, N, N-dimethylaniline, 1,8-diazobicyclo[5,4,0]undec-7-ene, pyridine, N-methylmorpholine, tetramethylethylenediamine, sodium carbonate, potassium carbonate, cesium carbonate, potassium tert-butoxide, sodium tert-butoxide, sodium methoxide or sodium ethoxide;
preferably, in step (1), the base is triethylamine.

6. The method according to claim 1, **characterized in that** in step (1), the solvent is a mixed solvent composed of any one or more of dichloromethane, trichloromethane, carbon tetrachloride, dichloroethane, acetonitrile, methanol, ethanol, isopropanol or tert-butanol;
preferably, in step (1), the solvent is a mixed solvent of dichloroethane and tert-butanol;
more preferably, in step (1), the volume ratio of dichloroethane to tert-butanol is 1:1.

7. The method according to claim 1, **characterized in that** in step (2), R² is selected from the group consisting of methyl or deuteromethyl; and/or, in step (2), the base is selected from the group consisting of triethylamine, diethylamine, N,N-diisopropylethylamine, triethylenediamine, 4-dimethylaminopyridine, N,N-dimethylaniline, 1,8-diazobicyclo[5,4,0]undec-7-ene, pyridine, N-methylmorpholine, tetramethylethylenediamine, sodium carbonate, potassium carbonate, cesium carbonate, potassium tert-butoxide, sodium tert-butoxide, sodium methoxide or sodium ethoxide; preferably, in step (2), the base is N,N-diisopropylethylamine.

8. The method according to claim 1, **characterized in that** in step (2), the solvent is a mixed solvent composed of any one or more of methanol, ethanol, isopropanol, acetonitrile, N,N-dimethylformamide, N,N-dimethylacetamide, N-methylpyrrolidone, or dimethylsulfoxide; preferably, in step (2), the solvent is N-methylpyrrolidone.

9. The method according to claim 1, **characterized in that** in step (3), the solvent used in the deprotection reaction is a mixed solvent composed of any one or more of water, tetrahydrofuran, dioxane, methanol, ethanol, isopropanol, or dichloromethane;
preferably, in step (3), the solvent used in the deprotection reaction is dioxane.

10. The method according to claim 1, **characterized in that** in step (3), the deprotection reagents used in the deprotection reaction are acids or bases;
preferably, in step (3), the deprotection reagents used in the deprotection reaction are selected from the group consisting of hydrochloric acid, trifluoroacetic acid, lithium hydroxide, sodium hydroxide or potassium hydroxide;
more preferably, in step (3), provided that R¹ is tert-butyl, the deprotection reagent used in the deprotection reaction is hydrochloric acid.

11. The method according to claim 1, **characterized in that** in step (4), R⁵ is selected from methyl or deuteromethyl; and/or, in step (4), the amide condensation reaction is carried out in the presence of a condensation agent, and the condensation agent is selected from the group consisting of isopropyl chloroformate, N,N'-carbonyldiimidazole, p-toluenesulfonyl chloride, (Boc)₂O, dicyclohexylcarbodiimide, diisopropylcarbodiimide, 1-(3-dimethylaminopropyl)-3-ethylcarbodiimide, *O*-(7-azabenzotriazol-1-yl)-bis(dimethylamino)carbenium hexafluorophosphate, *O*-(benzotriazol-1-yl)-bis(dimethylamino)carbenium hexafluorophosphate, O-(5-chlorobenzotriazol-1-yl)-bis(dimethylamino)carbenium hexafluorophosphate, *O-*(benzotriazol-1-yl)-bis(dimethylamino)carbenium tetrafluoroborate, *O*-(N-succinimide)-bis (dimethylamino)carbenium tetrafluoroborate, *O*-(*N*-endo-5-norcamphene-2,3-dicarbodiimide)-bis(dimethylamino)carbenium tetrafluoroborate, benzotriazol-1-yloxy-tri(dimethylamino) phosphonium hexafluorophosphate, benzotriazol-1-yloxy-tris(tetrahydropyrrolyl)phosphonium hexafluorophosphate, diphenylphosphoryl chloride, diethyl cyanophosphate, diphenylphosphoryl azide, thiodimethylphosphoryl azide, or bis(2-oxo-3-oxazolidinyl)phosphoryl chloride;
preferably, in step (4), the condensation agent is N,N'-carbonyldiimidazole;
more preferably, the molar ratio of compound (**V**) to the condensation agent is (1-5):(1-5); further preferably, the molar ratio of compound (**V**) to the condensation agent is 1:1.1.

12. The method according to claim 1, **characterized in that** in step (4), the solvent used for the amide condensation reaction is an aprotic solvent;
preferably, in step (4), the solvents used for the amide condensation reaction are selected from the group consisting of dichloromethane, dichloroethane, acetone, diethyl ether, diisopropyl ether, tetrahydrofuran, dioxane, toluene, acetonitrile, N,N-dimethylformamide, N,N-dimethylacetamide, or dimethyl sulfoxide;
more preferably, in step (4), the solvent used for the amide condensation reaction is dichloromethane.

13. The method according to claim 1, **characterized in that** compound (**III**) was prepared by the following method: 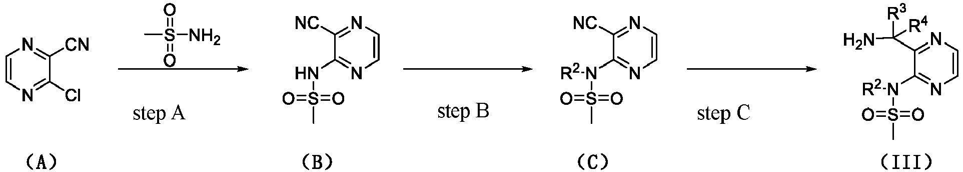
step A: In a solvent, compound (**B**) is synthesized by a substitution reaction of compound (A), as the starting material, with methanesulfonamide in the presence of a base;
step B: In a solvent, compound (**B**) reacts with a methylation reagent in the presence of a base, to obtain compound (**C**), wherein R² is selected from the group consisting of C₁-C₆ alkyl or C₁-C₆ deuterated alkyl
step C: compound (**III**) is prepared by reaction of compound (**C**) with a reducing reagent, wherein R³ and R⁴ are each independently selected from hydrogen or deuterium.

14. The method according to claim 13, **characterized in that** in step A, the molar ratio of compound (**A**), methane sulfonamide, and the base is (1-5): (1-5): (1-5);
and/or, in step B, the molar ratio of compound (**B**), the base, and the methylation reagent is (1-2): (1-5): (1-3);
preferably,
in step A, the molar ratio of compound (**A**), methane sulfonamide, and the base is 2:3:4;
and/or, in step B, the molar ratio of compound (**B**), the base, and the methylation reagent is 1.5:4.5:2.3.

15. The method according to claim 13, **characterized in that** in step A, the reaction temperature is ranged from -20 °C to 150 °C; and/or, in step B, the reaction temperature is ranged from -20 °C to 150 °C; and/or, in step C, the reaction temperature is ranged from -20 °C to 150 °C; preferably, in step A, the reaction temperature is ranged from 80 °C to 100 °C; and/or, in step B, the reaction temperature is ranged from 60 °C to 80 °C; and/or, in step C, the reaction temperature is ranged from 20 °C to 30 °C.

16. The method according to claim 13, **characterized in that** in step A, the base is selected from the group consisting of sodium carbonate, sodium bicarbonate, potassium carbonate, potassium bicarbonate, cesium carbonate, sodium hydroxide, potassium hydroxide, lithium hydroxide, calcium hydroxide, potassium tert-butoxide, sodium tert-butoxide, sodium methoxide, sodium ethoxide, triethylamine, diethylamine, N,N-diisopropylethylamine, triethylenediamine, 4-dimethylaminopyridine, N,N-dimethylaniline, 1,8-diazobicyclo[5,4,0]undec-7-ene, pyridine, N-methylmorpholine, or tetramethylethylenediamine;
preferably, the base is cesium carbonate.

17. The method according to claim 13, **characterized in that** in step A, the solvent is selected from the group consisting of methanol, ethanol, isopropanol, ethyl acetate, dichloromethane, acetonitrile, N,N-dimethylformamide, N,N-dimethylacetamide, dimethyl sulfoxide, N-methylpyrrolidone, tetrahydrofuran, toluene, dichloromethane, dioxane or water;
preferably, in step A, the solvent is acetonitrile.

18. The method according to claim 13, **characterized in that** in step B, R² is selected from methyl or deuteromethyl; and/or, in step B, the base used is selected from the group consisting of sodium carbonate, sodium bicarbonate, potassium carbonate, potassium bicarbonate, cesium carbonate, sodium hydroxide, potassium hydroxide, lithium hydroxide, calcium hydroxide, potassium tert-butoxide, sodium tert-butoxide, sodium methoxide, sodium ethoxide, triethylamine, diethylamine, N,N-diisopropylethylamine, triethylenediamine, 4-dimethylaminopyridine, N,N-dimethylaniline, 1,8-diazobicyclo[5,4,0]undec-7-ene, pyridine, N-methylmorpholine, or tetramethylethylenediamine;
preferably, the base is potassium carbonate.

19. The method according to claim 13, **characterized in that** in step B, the methylation reagents are selected from the group consisting of non-deuterated or deuterated methanol, iodomethane, dimethyl sulfate, methyl p-methylbenzenesulfonate, methyl p-nitrobenzenesulfonate, methyl trifluoromethanesulfonate, dimethyl carbonate, trimethyl phosphite, dimethyl phosphite, trimethyl phosphate, dimethyl phosphite, trimethyl orthoformate, trimethyl orthoacetate, N-methyl methanesulfonamide, and diazomethane;
preferably, in step B, the methylation reagents are iodomethane and deuterated iodomethane.

20. The method according to claim 13, **characterized in that** in step B, the solvent used is selected from the group consisting of methanol, ethanol, isopropanol, ethyl acetate, dichloromethane, acetonitrile, N,N-dimethylformamide, N,N-dimethylacetamide, dimethyl sulfoxide, N-methylpyrrolidone, tetrahydrofuran, toluene, dichloromethane, dioxane or water;
preferably, the solvent used is acetonitrile or N,N-dimethylformamide.

21. The method according to claim 13, **characterized in that** in step C, the reducing reagents used are selected from the group consisting of lithium aluminum hydride, diisobutyl aluminum hydride, sodium borohydride, lithium borohydride, zinc borohydride, sodium triacetoxyborohydride, sodium cyanoborohydride, lithium triethylborohydride, lithium triisobutylborohydride, lithium N,N-(dimethylamino)borohydride, dimethoxyethoxyaluminum hydride, bis(cyclopentadienyl)dicarbonyl titanium, borane, dimethyl sulfide borane, triethylsilane, zinc acetic acid, hydrogen or deuterium;
preferably, in step C, the reducing reagent used is hydrogen or deuterium.

22. The method according to claim 13, **characterized in that** in step C, a catalyst is used in the reaction, which is a transition metal catalyst;
preferably, in step C, the catalyst is Raney Ni, Pd/C, or Pt/C;
more preferably, in step C, the catalyst is Raney Ni.

23. The method according to claim 13, **characterized in that** in step C, the solvent used in the reaction is a mixed solvent composed of any one or more of methanol, ethanol, isopropanol, tert-butanol, diethyl ether, tetrahydrofuran, toluene, dichloromethane, dioxane or water;
preferably, in step C, the solvent used in the reaction is methanol.
